# EUROPEAN PATENT APPLICATION

(11) **EP 3 875 142 A1**
(43) Date of publication of application: **08.09.2021**
(21) Application number: 20160841.1
(22) Date of filing: 04.03.2020
(51) Int. Cl.: A61N 1/36

(54) **A NEUROMODULATION SYSTEM**

(71) Applicant: ONWARD Medical B.V., 5656 AE Eindhoven (NL)
(72) Inventor: CABAN, Miroslav, 1020 Renens (CH)
(74) Representative: DTS Patent- und Rechtsanwälte Schnekenbühl und Partner mbB

(57) **Abstract**

A neuromodulation system is disclosed for treating a subject with at least one abnormal body function and/or disease comprising the treatment and/or modulation of at least one condition and/or function and/or dysfunction of the subject's gastrointestinal system, cardiovascular system, sensory system, urinary system, respiratory system, reproductive system, thermoregulation system and/or locomotor system.

## Description

The present invention relates to the use of a neuromodulation system for treating a subject, in particular for treating a subject with abnormal body function and/or disease.

A wide variety of mental and/or physical processes are controlled or influenced by neural activity. Many problems or abnormalities with body functions can be caused by dysfunction, damage, disease and/or disorders in the nervous system and/or nerves. Effectively treating such abnormalities may be very difficult.

Neuromodulation systems have been developed for the treatment of dysfunction, damage, disease and/or disorders of the nervous system. One example are neuromodulation systems for the treatment of motoric dysfunction after spinal cord injury (SCI).

EP 2 868 343 A1 discloses a system to deliver adaptive electrical spinal cord stimulation to facilitate and restore locomotion after neuromotor impairment. Inter alia, a closed-loop system for real-time control of epidural electrical stimulation is disclosed, the system comprising means for applying to a subject neuromodulation with adjustable stimulation parameters, said means being operatively connected with a real-time monitoring component comprising sensors continuously acquiring feedback signals from said subject. The feedback signals provide features of motion of a subject, wherein the real-time monitoring component is operatively connected with a signal processing device receiving feedback signals and operating real-time automatic control algorithms. This known system improves consistency of walking in a subject with a neuromotor impairment. Reference is also made to Wenger N. et al., Closed-loop neuromodulation of spinal sensorimotor circuits controls refined locomotion after complete spinal cord injury, Science Translational Medicine, 6, 255 (2014).

EP 3 184 145 A1 discloses systems for selective spatiotemporal electrical neurostimulation of the spinal cord. A signal processing device receiving signals from a subject and operating signal-processing algorithms to elaborate stimulation parameter settings is operatively connected with an Implantable Pulse Generator (IPG) receiving stimulation parameter settings from said signal processing device and able to simultaneously deliver independent current or voltage pulses to one or more multiple electrode arrays. The electrode arrays are operatively connected with one or more multi-electrode arrays suitable to cover at least a portion of the spinal cord of said subject for applying a selective spatiotemporal stimulation of the spinal circuits and/or dorsal roots, wherein the IPG is operatively connected with one or more multi-electrode arrays to provide a multipolar stimulation. Such system allows achieving effective control of locomotor functions in a subject in need thereof by stimulating the spinal cord, in particular the dorsal roots, with spatiotemporal selectivity.

However, neuromodulation systems may additionally and/or alternatively be applied for the treatment of dysfunctions, damages and/or disorders of the nervous system of a patient suffering from spinal cord injury and/or any other disease.

Surprisingly, it has recently been found that neuromodulation systems may be used for the treatment of autonomic dysfunction after SCI.

Neuromodulation systems may be also used for the treatment of symptoms occurring in other diseases.

It is an object of the present invention to improve the use of a neuromodulation system for a subject with at least one impaired body function.

This object is solved according to the present invention by the use of a neuromodulation system for treating a subject, with the features of claim 1. Accordingly, the use of a neuromodulation system for treating a subject with at least one abnormal body function and/or disease comprising the treatment and/or modulation of at least one condition and/or function and/or dysfunction of the subject's gastrointestinal system, cardiovascular system, sensory system, urinary system, respiratory system, reproductive system, thermoregulation system and/or locomotor system. In particular, the specific use of a neuromodulation system for treating a subject may enable that the neuromodulation provided by a neuromodulation system is specifically adapted to the subject's needs and/or impairment and/or rehabilitation status and/or environment. This may enable advancing progress in rehabilitation and thus a most efficient and fast rehabilitation process.

Abnormal body function may be or may comprise any type of pathophysiological condition.

In general, the gastrointestinal system of the subject may comprise the upper gastrointestinal tract and/or the lower gastrointestinal tract. In particular, the gastrointestinal system may comprise the mouth, esophagus, stomach and/or intestine of the subject. In particular, the intestine may comprise the small intestine (duodenum, jejunum, ileum) and/or the large intestine (cecum, appendix, ascending colon, right colic flexure, transverse colon, left colic flexure, descending colon, sigmoid colon, rectum and/or anus). In general, sphincters of the respective organs may be included in the gastrointestinal system.

Further, the gastrointestinal system of the subject may comprise the liver, gallbladder, pancreas, pancreatic duct and/or immune system of the gastrointestinal system of the subject.

The reproductive system of the subject may comprise testes, scrotum, prostate, penis, urethra, vas deferens, Cowper's gland, vulva, ovaries, breasts and/or cervix of the subject.

The urinary system of the subject may comprise kidney(s), renal pelvis, ureter, urinary bladder, urethra and/or adrenal gland(s) of the subject.

The cardiovascular system of the subject may comprise arteries, capillaries, veins, portal veins, the heart and/or coronary vessels.

The respiratory system of the subject may comprise nose, nasal cavities, sinuses, pharynx, larynx (at least partially), trachea, lung, bronchi, bronchioles and/or alveoli.

The sensory system of the subject may comprise sensory neurons, sensory receptor cells, neural pathways and/or parts of the brain involved in sensory perception.

The locomotor system of the subject may comprise the skeleton of the subject and/or at least one muscle, nerve, bone, cartilage and/or tendon. In particular, the locomotor system may comprise upper and lower body parts, including arms, legs, trunk, abdomen, neck, head, etc.

The thermoregulation system of the subject may comprise sweat glands, vascularization (in particular skin vascularization), adipose tissue (in particular white adipose tissue and/or brown adipose tissue), muscles, central nervous system (e.g. Lissauer's tract, spinal cord, brain, e.g. hypothalamus, thalamus) and/or thermoreceptors.

The neuromodulation system may be or may comprise at least one of a neuromodulator, a controller, a processor, a sensor, a communication module, a telemetry module and/or a storage means. In particular, the neuromodulator may be a neurostimulator.

The neurostimulator may be or may comprise a pulse generator, e.g. an implantable pulse generator and/or non-implantable pulse generator, a lead and/or at least one electrode.

The at least one condition and/or function and/or dysfunction may comprise at least one of acetylcholine dysregulation, acidity of stomach, autonomic dysreflexia, back pain, sphincter function, blood volume dysregulation, breathing deficiency, bronchial tree obstruction, cardiac activity, cholesterol imbalance, motor deficits, mucosa renewal, immune system deficit, immobility of the stomach and/or intestine and/or colon, lipogenesis/lipolysis imbalance, lower limb paralysis and/or upper limb paralysis (bilateral and/or unilateral), detrusor muscle, bronchial deficiency, alveolar deficiency, mean venous pressure, mean arterial pressure, mucous layer of stomach, erectile dysfunction, neck stabilization, odour, hypotension, orthostatic hypotension, hypertension, salivary glands function, supraspinal cardiovascular regulation, swallowing, taste, thermoregulation, trunk stabilization, vision, cutaneous vascularization, sweat glands, lipid metabolism and/or glycogen metabolism. In particular, the specific use of a neuromodulation system for treating at least one of these conditions and/or functions and/or dysfunctions may enable that physiological deficits of an impaired subject are at least partially restored. In particular, this may enable that the subject's performance and/or quality of live is enhanced.

In particular, motor deficits may be or may comprise any deficits of the motor function of the upper and/or lower extremities, and/or the trunk and/or abdomen and/or neck and/or head.

In particular, mucosa renewal may comprise mucosa renewal of the intestine, colon and/or stomach.

In particular, a sphincter may be or may comprise any autonomous and/or voluntary regulated sphincter. Sphincter may refer to the upper and/or lower sphincter of the esophagus, pyloric, ileocecal, urethral (lower urinary and/or upper urinary sphincter) and/or internal/external anal sphincter.

Sphincter dysfunction may be or may comprise dysfunction of the upper and/or lower sphincter of the esophagus, pyloric, ileocecal, urethral and/or internal/external anal sphincter.

In particular, the at least one condition may comprise at least one function and/or dysfunction.

The at least one abnormal body function and/or disease may be or may comprise myasthenia, dystonia, epilepsy, muscular hyperfatigability, ulcers, Helicobacter Pylori infection, inflammatory bowel disease, Crohn's disease, ulcerative colitis, irritable bowel syndrome, spinal cord injury, incontinence, hypertension, hypotension, sudden episodic high blood pressure, dyspnea, chronic obstructive pulmonary disease, cardiac failure, cardiac dysrhythmia, bradycardia, tachycardia, cardiac arrest, impairment of genital plexus (e.g. after prostate removal), HIV infection, herniated disk, tumor, obesity, gastroesophageal reflux disease, lower esophageal sphincter failure, achalasia, amyotrophic lateral sclerosis, trauma, stroke, damaged dorsal root, pernicious anemia, vitamin D deficiency, multiple sclerosis, diabetes mellitus, autoimmune disease, Sjogren syndrome, Mikulicz syndrome, loss of sympathetic control, dysphagia, overreactive bladder syndrome, hyperhomocysteinemia, neurodegenerative disease, Parkinson's disease, Alzheimer's disease, essential tremor, schizophrenia, osteoporosis and/or hyperthyroidism. In particular, the specific use of a neuromodulation system for treating a subject at least one of these diseases and/or abnormal functions may enable that the symptoms of these abnormal functions and/or diseases may be at least partially cured and/or symptoms may be relieved during stimulation. In particular, this may enable that the subject's performance and/or quality of live is enhanced.

In particular, incontinence may be or may comprise fecal and/or urinary incontinence.

In particular, neurodegenerative disease may be or may comprise Alzheimer's disease and/or Parkinson's disease.

In particular, treating a subject may be or may comprise treating at least one of muscular cholinergic synapse, stomach, sympathetic and/or parasympathetic nervous system, heart, cardiac muscle, spinal cord, lower urinary sphincter, autonomous and/or voluntary regulated sphincters, Renin Angiotensin system, kidney, lower bowel sphincter, diaphragm, intercostal muscle, bronchia, smooth muscles of the pulmonary system, solitary tract, sinoatrial/atrioventricular nodes, pancreas, genital plexus, dorsal root to muscle (e.g. pharynx), colon, vago-vagal reflex, cajal cell, muscle, smooth muscle, nerve, concerned nerve or group of nerves, concerned plexus (including but not limited to genital, Auerbach, Meissner, cardiac, pulmonary, cervical, lombal, sacral and/or coeliaque plexus), liver, adrenal gland, lower esophageal sphincter, alveoli, pulmonary circulation, associated cortex and/or regulatory center, salivary gland and/or detrusor muscle. In other words, a more or less specific neuromodulation may be enabled.

In particular, these targets could be treated directly and/or indirectly. In other words, the at least one target may be directly the place of dysfunction and/or a way to modulate the dysfunction.

In particular, conditions could be modulated by targeting these targets.

In particular, intercostal muscles may include innermost, internal and/or external intercostal muscles.

In particular, intercostal muscles may include innermost, internal and/or external intercostal muscles.

Respiratory tractus may include respiratory system and/or associated blood circulation and/or smooth muscles.

Spinal cord may include dorsal roots, afferent and/or efferent nerves, ganglions.

The use of the neuromodulation system according to the present invention may comprise the use of a neuromodulation system throughout rehabilitation of the subject and/or during at least one stage of rehabilitation. In particular, the stage of rehabilitation may be or may comprise at least one of stabilization in bed, mobilization in bed, mobilization at bedside, verticalization, blood pressure lowering, blood pressure rehabilitation, blood pressure stabilization, cardiac rehabilitation, cardiac stabilization, standing, stepping, balance training, locomotor training, fluent locomotion, pre-surgery, post-surgery, pre-implantation, post-implantation, post-setting of neuromodulation system, spinal shock and/or daily life activities. In particular, the use of the neuromodulation system may comprise the specific use of the neuromodulation system during least one specific rehabilitation stage. This may enable best possible treatment during each stage and/or phase of rehabilitation.

It is generally possible that the use of the neuromodulation system is specifically adapted to the subject's individual needs and/or preferences and/or rehabilitation stage. It is further generally possible, that the use of the neuromodulation system is altered throughout rehabilitation.

Further, the use of the neuromodulation system may comprise or may be combined with the use of an assistive device, wherein the assistive device may be or may comprise at least one of a breathing device, pulling handles, cables from ceiling, aquatic equipment, a treadmill, an exoskeleton, an orthosis, a robot, a bike, a breather, crutches, a cuff, a cuff for functional electrical stimulation, a smartwatch, a motorized mattress, non-invasive ventilation, a standing frame, a rollator, a body weight support system, elastic bands, weights, a slide board, a step machine, a trike, a walker, a robot-physiotherapist, balls and/or half-balls. In particular, the use of an assistive device together with a neuromodulation system and/or as part of a neuromodulation system may enable most efficient progress in rehabilitation.

Aquatic equipment may be or may comprise at least on of noodles, floating vests, boards, etc.

The neuromodulation system may be neurostimulation system, wherein the neurostimulation system may be or may comprise an electrical neurostimulation system and/or mechanical neurostimulation system and/or pharmacological neurostimulation system. It may be possible that the pharmacological neurostimulation system comprises a drug pump.

Alternatively, the neurostimulation system may be or may comprise an optical stimulation system, an ultrasound stimulation system and/or a magnetic field stimulation system.

In particular, an electrical stimulation system may be or may comprise a pulse generating system. In particular, the pulse generating system may be an implantable pulse generating system (IPG). The electrical stimulation system may alternatively and/or additionally comprise an electrode array comprising multiple electrodes and/or a lead.

In particular, the neuromodulation system may be or may comprise at least one of a transcutaneous electrical stimulation system (TENS), epidural electrical stimulation system (EES), functional electrical stimulation system (FES), intramuscular stimulation system (IMS), dorsal root ganglion stimulation system, subdural stimulation system, cardiac stimulation system, optogenetics, optotrodes, patch-clamp, intra-cellular electrodes and/or extra-cellular electrodes. It may be generally possible that the neuromodulation system is a central nervous system (CNS) stimulation system and/or peripheral nervous system stimulation (PNS) system.

In particular, for the use of a neuromodulation system for stimulation of the CNS may comprise that neurostimulation may be applied to any area of the brain and/or spinal cord (including but not limited to spinal nerves, ganglions, nerve roots) and/or nervous plexus and/or glands related to the nervous system (e.g. medulla). In particular, electrical neurostimulation may be applied to at least one of T6 - T9, T6 or higher, T2-L1, L1-L2, S2-S4, T10-L1, T10-L2 and S2-S4, C3-C5, T1-T5, T5-T9, L5-S5, L2-S1, L1-S1, T7-T12, T5-T8, L2-S2, T1-T6, T1-T5, T6-T9, C1-C3, above C1, C1-C4, C1-C4 and above, N.X, N. IX, C1-C5, T7, hypothalamus, medulla, T7-L1, C1-S1, L3-S4.

Epidural Electrical Stimulation (EES) is known to restore motor control in animal and human models and has more particularly been shown to restore locomotion after spinal cord injury by artificially activating the neural networks responsible for locomotion below the spinal cord lesion (Capogrosso, M., et al., A Computational Model for Epidural Electrical Stimulation of Spinal Sensorimotor Circuits, Journal of Neuroscience , 33 (49), 19326-19340 (2013); Courtine G., et al., Transformation of nonfunctional spinal circuits into functional states after the loss of brain input, Nat Neurosci. 12(10), 1333-1342 (2009); Moraud EM., et al, Mechanisms Underlying the Neuromodulation of Spinal Circuits for Correcting Gait and Balance Deficits after Spinal Cord Injury, Neuron, 89(4), 814-828 (2016)). EES does not directly stimulate motor-neurons but the afferent sensory neurons prior to entering into the spinal cord. In this way, the spinal networks responsible for locomotion are recruited indirectly via those afferents, restoring globally the locomotion movement by activating the required muscle synergies.

Peripheral Nervous System (PNS) Stimulation systems used to date in the clinic are known as Functional Electrical Stimulation (FES) that provides electrical stimulation to target muscles with surface electrodes, either directly through stimulation of their motor fibers (neuro-muscular stimulation), or through a limited set reflexes (practically limited to the withdrawal reflex) or by transcutaneously stimulating the peripheral nerves. The resulting muscle fatigue has rendered FES unsuitable for use in daily life. Furthermore, successes have remained limited through cumbersome setups when using surface muscle stimulation, unmet needs in terms of selectivity (when using transcutaneous nerve stimulation) and a lack of stability (impossible to reproduce exact electrode placement on a daily basis when stimulating muscles, moving electrodes due to clothes, sweating).

The neuromodulation system may be an open-loop system and/or closed-loop system and/or pseudo open-loop system.

In particular, an open-loop system may be operated manually, with a simple design. Thus, open loop systems may be stable and economical compared to closed loop systems.

On the contrary, a closed-loop system may be accurate even in the presence of nonlinearities. The sensitivity of the system may be made small to make the system more stable. The closed loop system may be less affected by noise.

In particular, the closed-loop system may comprise at least one sensor. In particular, this may enable that the patient's status and/or performance may be assessed and/or provided to the neuromodulation system. This may further enable that the neuromodulations performance is specifically adapted to the patient's needs.

A sensor may be at least one of an accelerometer, pressure sensor, motion capture sensor, force plate, rehabilitation device, electrode, electrode lead, thermometer (e.g. nose thermometer, skin thermometer, implanted thermometer), infrared camera, doppler ultrasound, electrocardiogram, GPS, smartphone, smartwatch, action-camcorder, video processing, virtual reality module, magnetic resonance imaging, plethysmography sensor, pulse oximetry sensor, pulse pressure sensors, inertial measurement unit (IMU), goniometer, insole, electroencephalography (EEG), skin resistance sensor, infrared sensor, spirometer (with or without dilution), stethoscope, tensiometer, intestinal motility sensor, bowel sensor and/or pH sensor.

The use of the neurostimulation system may comprise and/or may be combined with the use of a feedback system, wherein the feedback system is an audio and/or visual and/or sensory-motoric feedback system. In particular, this may enable that the subject is provided with feedback information including e.g. the neuromodulation system's performance and/or the patient's performance and/or patient's body reaction(s) in order to advance rehabilitation.

In particular, it may be possible that the feedback system is part of the closed-loop system.

In particular, the audio feedback system may comprise e.g. headphones, speakers, providing sound or voice signals.

The visual feedback system may comprise e.g. floor projection, goggles, LEDs and/or lights, and/or a screen for providing visual signals.

The sensory-motoric feedback system may provide and/or comprise signals such as haptic signals, pain, paresthesia, signals provided by a person, sensory pathways stimulation, temperature signals and/or changes, touch, vibration etc.

The feedback system may be part of the closed-loop system and/or vice versa.

The at least one sensor may provide quality and/or performance metrics of the use of the neuromodulation system. The quality and/or performance metrics may be or may comprise sensitivity scores, motor scores, quality of movement scores, performance and/or activity, participation, reaction times, clinical assessment scores, autonomous system scores. In particular, the quality and/or performance scores may be or may comprise autonomous muscular reflexes and/or stretch reflexes, fluidity, blood O2 saturation, blood speed, distance to path ratio, e.g. cardiac parameters, gait parameters, heart rate, force, speed, distance, stress level, muscle strength, range of motion, pulse pressure wave, QoL questionnaires, quantity and timing of urine, respiratory rate, respiratory volume and/or stress markers, e.g. in body fluids.

Further, the system may provide tonic and/or burst and/or high-frequency neuromodulation.

Tonic neuromodulation may be applied with 1 to 125 Hz.

Burst neuromodulation may be applied by applying pairs, triplets, quadruplets, quintuplets of pulse at burst frequency of at least 200Hz, preferably 300 to 750 Hz, at an interburst frequency of 1 to 125 Hz, preferably 10 to 60Hz.

High frequency neuromodulation may be applied with 200 Hz to 10 kHz.

The system may provide frequency neuromodulation on top of a carrying frequency. In particular, the carrying frequency may be applied with 1 to 10 kHz, preferably 5 to 10 kHz.

In general, indoor and/or outdoor use of the neuromodulation system may be possible. Further, the use of the system may be possible during activities performed by the subject. In particular, this may enable use of the neuromodulation system during daily life activities and/or sports (including e.g. walking, running, cycling, hiking, horse riding, skiing, swimming, sailing, jumping, ball sports, dancing), etc.

Indoor use may comprise use in least one of clinics, gym, physiotherapy room, occupational room, aquatic room, sport activity room, recreation facility, restaurant, cinema, bathroom, bedroom, kitchen, living room, etc.

Outdoor use may comprise use on streets (including gravel, concrete pavement, cobble stone, rocky hiking trails, etc.), grass, shallow water, sand and/or snow.

It is generally possible that the neuromodulation system may be used and/or operated by at least one robot and/or a person. In particular, the robot may be an assistive robot for healthcare and/or a humanoid robot. In particular, the person may be a family member, a friend, a therapist, a physiotherapist, a nurse, an electrophysiologist, a non-professional user, a professional user, an occupational therapist, a surgeon, a technician (clinical/ FCE), and/or a (treating) physician of the subject and/or the subject himself. Further, the neuromodulation system may be used by a virtual assistant. Additionally, and/or alternatively, the neuromodulation system may be operated locally or remotely.

The use of the neuromodulation system may comprise the use of a network and/or device and/or platform. In particular, the use of the neuromodulation system may comprise the use of at least one of a cloud, game console, internet, local database, local network, medical and/or rehabilitation device, smartphone, smartwatch and/or social media.

In particular, the use of the neuromodulation system may be performed to perform a task including but not limited to balancing, bending over, coughing, walking, climbing, climbing stairs, swimming and/or performing other tasks under water, sitting, sitting on ground, sit-to-stand, grasping, squatting, standing, swallowing, erecting, inclined waking, neck training, posture training, normal neck posture, normal gait, normal intestinal movement during digestion, normal trunk posture, (normal (use of at least one affected limb, (normal) use of lower limbs, prevention of muscle weakness, regulation of stomach acidity and mucosal layer, respiration, transferring, voluntary regulated sphincter action, zero-G-training and/or performing above-mentioned tasks in zero-G.

The use of the neuromodulation system may be performed to evoke responses such as but not limited to cardiac response, hormonal response, motor response, neurotransmitter response, sensory response, smooth muscle contraction, smooth muscle dilatation, general somatic afferent response (perception of touch, pain and/or temperature and/or others), general somatic efferent response (voluntary motor innervation and/or others), general visceral efferent response (motor innervation to smooth muscles and/or heart muscle and/or glands), special afferent response (visceral: smell, taste, somatic: vision, hearing, balance), respiration, cough and/or erection.

Further details and advantages shall now be disclosed by specific embodiments.

### Gastrointestinal system

In a preferred embodiment, the use of a neuromodulation system for treating a subject with at least one abnormal body function and/or disease comprises the treatment and/or modulation of at least one condition and/or function and/or dysfunction of the subject's gastrointestinal system.

In this embodiment, the at least one condition and/or function and/or dysfunction comprises bowel sphincter function.

Alternatively, and/or additionally, the at least one condition and/or function could be or could comprise at least one of acidity of stomach, cholesterol imbalance, lipogenesis/lipolysis imbalance, mucosa renewal, intestinal immobility, lower esophageal sphincter function, salivary glands function, mucous layer of stomach function, swallowing, taste, immune system deficit, lipid metabolism and/or glycogen metabolism.

In this embodiment, the at least one abnormal body function and/or disease is incontinence.

However, in an alternative embodiment, the abnormal body and/or disease could be or could comprise ulcers, Helicobacter Pylori infection, inflammatory bowel disease, Crohn's disease, ulcerative colitis, irritable bowel syndrome, tumor, gastroesophageal reflux disease, lower esophageal sphincter failure, achalasia, trauma, spinal cord injury, stroke, tumor, pernicious anemia, vitamin D deficiency, Sjogren syndrome, Mikulicz syndrome, dysphagia, diabetes and/or obesity.

In this embodiment, treating the subject comprises treating the subject's lower bowel sphincter.

In this embodiment, treating the subject comprises treating a voluntary regulated sphincter of the subject.

Alternatively and/or additionally, treating the subject could be or could comprise treating at least one of stomach, autonomous regulated sphincter, pancreas, dorsal root to muscle (e.g. pharynx), circulatory cholesterol uptake, colon, vago-vagal reflex, cajal cell, smooth muscle, nerve, Auerbach plexus, Meissner plexus, lower esophageal sphincter, liver, pancreas and/or salivary gland.

In this embodiment, the use of the neuromodulation system is performed throughout rehabilitation of the subject.

In an alternative embodiment, the use of the neuromodulation system could be performed alternatively during at least one stage of rehabilitation. The stage of rehabilitation could be or could comprise at least one of stabilization in bed, mobilization in bed, mobilization at bedside, verticalization, pre-surgery, post-surgery, pre-implantation, post-implantation, spinal shock and/or daily life activities.

In this embodiment, the use of the neuromodulation system is performed to perform voluntary regulated sphincter action.

In an alternative embodiment, a task to be performed may additionally and/or alternatively include normal intestinal movement during digestion, regulation of stomach acidity and/or mucosal layer, eating, swallowing and/or drinking.

In general, indoor and/or outdoor use of the neuromodulation system is possible.

Further, the use of the system is possible during activities performed by the subject.

In this embodiment, the neuromodulation system is a neurostimulation system, wherein the neurostimulation system comprises an electrical neurostimulation system.

However, alternatively and/or additionally, a mechanical neurostimulation system and/or pharmacological neurostimulation system could be generally possible.

In this embodiment, the electrical neurostimulation system is an intramuscular stimulation system.

In an alternative embodiment, the electrical neurostimulation system could be or could comprise at least one of a transcutaneous electrical stimulation system, epidural electrical stimulation system, functional electrical stimulation system, dorsal root ganglion stimulation system, subdural stimulation system, optogenetics, optotrodes, patch-clamp, intra-cellular electrodes and/or extra-cellular electrodes.

For treating ulcers, Helicobacter pylori infection, Crohn's disease, irritable bowel syndrome and/or pernicious anemia, an electrical stimulation system (e.g. epidural and/or subdural stimulation system) could be used to provide stimulation at least partially to the level of T6-T9 and/or L1-S1 of the subject.

For treating incontinence (e.g. after spinal cord injury), an electrical stimulation system (e.g. epidural and/or subdural stimulation system) could be used to provide stimulation at least partially to the level of T10-L2 and/or S2 and/or S4 of the subject.

For treating diabetes mellitus, an electrical stimulation system (e.g. epidural and/or subdural stimulation system) could be used to provide stimulation at least partially to the level of T7-T12 and/or T5-T8 of the subject.

For treating gastroesophageal reflux disease and/or achalasia, an electrical stimulation system (e.g. epidural and/or subdural stimulation system) could be used to provide stimulation at least partially to N.X.

For treating pernicious anemia, vitamin D deficiency, diabetes mellitus, Sjogren syndrome, and/or Mikulicz syndrome the electrical stimulation system (e.g. epidural and/or subdural stimulation system) could be used to provide stimulation at least partially to the level of C1-C4 of the subject.

For treating dysphagia (e.g. after spinal cord injury), an electrical stimulation system (e.g. epidural and/or subdural stimulation system) could be used to provide stimulation at least partially to N.X., N.IX and/or the level of C1-C5 of the subject.

The use of the neuromodulation system could be performed to evoke responses such as defecation.

In this embodiment, the neuromodulation system is an open-loop system.

However, alternatively, a closed-loop system and/or pseudo open-loop system could be generally possible.

A sensor of a closed-loop neuromodulation device could be or could comprise at least one of intestinal motility sensor, bowel sensor, pH sensor and/or another sensor.

In this embodiment, the use of the neuromodulation system comprises the use of the neuromodulation system post-setting the neuromodulation system.

Alternatively, and/or additionally, the use of the neuromodulation system could comprise the use of the neuromodulation system throughout rehabilitation of the subject and/or during at least one stage of stabilization in bed, mobilization in bed, mobilization at bedside, verticalization, surgery, post-surgery, pre-implantation, post-implantation, spinal shock and/or daily life activities.

In this embodiment, the system is used indoor.

In this embodiment, the indoor use comprises the use in clinics.

Alternatively, and/or additionally, the indoor use could comprise the use in at least one of gym, physiotherapy room, occupational room, aquatic room, sport activity room, recreation facility, restaurant, cinema, bathroom, bedroom, kitchen, living room, etc.

Alternatively, and/or additionally outdoor use of the neuromodulation system could be possible.

Further, the use of the system is generally possible during activities performed by the subject.

In this embodiment, the neuromodulation system is used and/or operated by at least one person.

In this embodiment, the neuromodulation system is used by the subject himself.

In general, any other person, such as a family member, a friend, a therapist, a physiotherapist, a nurse, an electrophysiologist, a non-professional user, a professional user, an occupational therapist, a surgeon, a technician (clinical/ FCE) and/or a (treating) physician of the subject could use the system.

Further, the neuromodulation system could be used by a robot.

### Reproductive system

In another preferred embodiment, the use of a neuromodulation for treating a subject with at least one abnormal body function and/or disease comprises the treatment and/or modulation of at least one condition and/or function and/or dysfunction of the subject's reproductive system.

In particular, in this embodiment, the at least one condition and/or function and/or dysfunction is erectile dysfunction.

In this embodiment, the at least one abnormal body function and/or disease comprises impairment of genital plexus after prostate removal.

In an alternative embodiment, the at least one abnormal body function and/or disease could additionally and/or alternatively comprise at least one of stroke, trauma, spinal cord injury, tumor, diabetes, Parkinson's disease, Alzheimer's disease and/or neurodegenerative disease.

In this embodiment, treating the subject comprises treating the genital plexus, smooth muscles and nerves of the subject.

However, in an alternative embodiment, treating the subject could comprise treating the genital plexus and/or smooth muscles and/or nerves of the subject.

In this embodiment, the use of the neuromodulation system is performed throughout rehabilitation of the subject.

In an alternative embodiment, the use of the neuromodulation system could be performed during at least one stage of rehabilitation.

The stage of rehabilitation could be or could comprise at least one of stabilization in bed, mobilization in bed, mobilization at bedside, verticalization, blood pressure lowering, blood pressure rehabilitation, cardiac rehabilitation, standing, stepping, balance training, locomotor training, fluent locomotion, pre-surgery, post-surgery, post-implantation, post-setting of neuromodulation system, spinal shock and/or daily life activities.

In this embodiment, the neuromodulation system is a neurostimulation system, wherein the neurostimulation system comprises an electrical neurostimulation system.

However, alternatively and/or additionally, a mechanical neurostimulation system and/or pharmacological neurostimulation system could be generally possible.

In this embodiment, the electrical neurostimulation system is an epidural electrical stimulation system.

In this embodiment, the epidural electrical stimulation system is used to provide stimulation at least partially to level L5-S5 of the subject.

In an alternative embodiment, the stimulation system could be alternatively and/or additionally at least one of a transcutaneous electrical stimulation system, functional electrical stimulation system, intramuscular stimulation system, dorsal root ganglion stimulation system, subdural stimulation system, optogenetics, optotrodes, patch-clamp, intra-cellular electrodes and/or extra-cellular electrodes.

In this embodiment, the use of the neuromodulation system is performed to evoke responses such as erection.

However, in general, any other type of response, such as motor and/or sensory response of the reproductive system could be generally possible.

In this embodiment, the neuromodulation system may be an open-loop system.

However, alternatively, a closed-loop system and/or pseudo open-loop system could be generally possible.

In this embodiment, the use of the neuromodulation system comprises the use of burst neuromodulation, in particular, burst neurostimulation.

In general, burst neuromodulation could be applied by applying pairs, triplets, quadruplets, quintuplets of pulse at burst frequency of at least 200Hz, preferably 300 to 750 Hz, at an interburst frequency of 1 to 125 Hz, preferably 10 to 60Hz.

However, alternatively and/or additionally, the use of tonic and/or high-frequency neuromodulation, in particular neurostimulation could be generally possible.

The system could provide frequency neuromodulation on top of a carrying frequency. In particular, the carrying frequency could be applied with 1 to 10 kHz, preferably 5 to 10 kHz.

In this embodiment, the use of the neuromodulation system could comprise the use of an assistive device.

However, alternatively, the use of the neuromodulation system in this embodiment could not comprise the use of an assistive device.

### Urinary system

In a further preferred embodiment, the use of a neuromodulation system for treating a subject with at least one abnormal body function and/or disease comprises the treatment and/or modulation of at least one condition and/or function and/or dysfunction of the subject's urinary system.

In this embodiment, the at least one condition and/or function and/or dysfunction is bladder sphincter function.

Specifically, bladder sphincter function can be understood as bladder sphincter dysfunction.

In an alternative embodiment, the at least one condition and/or function and/or dysfunction could additionally and/or alternatively comprise at least one of blood volume, mean arterial pressure, vesical sphincter and/or others.

In this embodiment, the at least one abnormal body function and/or disease is incontinence.

However, in an alternative embodiment, the at least one abnormal body function and/or disease could be or could comprise hypertension, hypotension, sudden episodic high blood pressure, trauma, spinal cord injury, stroke, tumor, damaged dorsal root, Parkinson's disease, Alzheimer's disease, neurodegenerative disease, overreactive bladder syndrome and/or others.

In this embodiment, treating the subject comprises treating a muscle (muscles) of the subject.

In this embodiment, treating the subject comprises treating the lower urinary sphincter of the subject.

Alternatively, and/or additionally, at least one of sympathetic and/or parasympathetic nervous system, autonomous and/or voluntary regulated sphincters, smooth muscles, nerves, kidney, detrusor, kidney and/or others could be treated.

The use of the neuromodulation system according to this embodiment comprises the use of a neuromodulation system throughout rehabilitation of the subject.

Alternatively, and/or additionally, the use of a neuromodulation system could comprise the use of a neuromodulation system during at least one stage of rehabilitation.

In particular, the stage of rehabilitation could be or could comprise at least one of stabilization in bed, mobilization in bed, mobilization at bedside, verticalization, blood pressure lowering, blood pressure rehabilitation, cardiac rehabilitation, standing, stepping, balance training, locomotor training, fluent locomotion, pre-surgery, post-surgery, pre-implantation, post-implantation, post-setting of neuromodulation system, spinal shock and/or daily life activities.

In this embodiment, the neuromodulation system is a neurostimulation system, wherein the neurostimulation system comprises an electrical neurostimulation system.

However, alternatively and/or additionally, a mechanical neurostimulation system and/or pharmacological neurostimulation system could be generally possible.

In this embodiment, the electrical neurostimulation system is an epidural electrical stimulation system.

In this embodiment, the epidural electrical stimulation system is used to provide stimulation at least partially to level L1-L2 and/or S2-S4 of the subject.

Alternatively, the epidural electrical stimulation system could be used to provide stimulation at least partially to level L3-L4 of the subject.

In an alternative embodiment, the stimulation system could be alternatively and/or additionally at least one of a transcutaneous electrical stimulation system, functional electrical stimulation system, intramuscular stimulation system, dorsal root ganglion stimulation system, subdural stimulation system (also for the treatment of overreactive bladder syndrome), optogenetics, optotrodes, patch-clamp, intra-cellular electrodes and/or extra-cellular electrodes.

For treating hypotension and/or hypertension, the electrical stimulation system (e.g. epidural and/or subdural stimulation system) could be used to provide stimulation at least partially to level T10-L1 of the subject.

In general, indoor and/or outdoor use of the neuromodulation system is possible in this embodiment.

Further, the use of the system is possible during activities performed by the subject.

In this embodiment, the neuromodulation system is a closed-loop system.

However, alternatively, an open-loop system and/or pseudo open-loop system could be generally possible.

In this embodiment, the use of the neuromodulation system comprises the use of tonic neuromodulation, in particular tonic neurostimulation.

In this embodiment, tonic neuromodulation could be applied with 1 to 125 Hz.

It could be possible that the system could provide frequency neuromodulation on top of a carrying frequency. In particular, the carrying frequency could be applied with 1 to 10 kHz, preferably 5 to 10 kHz.

However, alternatively and/or additionally, the use of burst and/or high-frequency neuromodulation, in particular neurostimulation could be generally possible.

The closed-loop system comprises a sensor.

In general, a sensor of the closed-loop neuromodulation system could be or could comprise at least one of a tensiometer and/or electrocardiogram and/or any other type of sensor.

It is generally possible that the neuromodulation system is used and/or operated by at least one person.

In particular, the person may be a family member, a friend, a therapist, a physiotherapist, a nurse, an electrophysiologist, a non-professional user, a professional user, an occupational therapist, a surgeon, a technician (clinical/ FCE), and/or a (treating) physician of the subject.

Further, the neuromodulation system could be used by a robot.

The use of the neuromodulation system in this embodiment could comprise the use of an assistive device.

However, alternatively, the use of the neuromodulation system in this embodiment could not comprise the use of an assistive device.

In this embodiment, the use of the neuromodulation system is performed to evoke a response of the sphincter (contraction).

### Cardiovascular system

In a further preferred embodiment, the use of a neuromodulation system for treating a subject with at least one abnormal body function and/or disease comprises the treatment and/or modulation of at least one condition and/or function and/or dysfunction of the subject's cardiovascular system.

In this embodiment, the at least one function and/or dysfunction comprises mean venous pressure and mean arterial pressure.

However, in an alternative embodiment, the at least one condition and/or function and/or dysfunction could comprise at least one of cardiac activity, supraspinal cardiovascular regulation, autonomic dysreflexia, orthostatic hypotension and/or mean venous pressure or mean arterial pressure.

In this embodiment, the abnormal body function is hypertonia.

In an alternative embodiment, the abnormal body function and/or disease could be or could comprise dystonia, spinal cord injury, hypotension, sudden episodic high blood pressure, supraspinal cardiovascular regulation, cardiac failure, cardiac dysrhythmia, bradycardia, tachycardia, cardiac arrest, tumor, trauma and/or stroke.

In this embodiment, treating the subject comprises treating nerves of the subject.

In this embodiment, treating the subject comprises treating the spinal cord of the subject.

In this embodiment, the treatment of the subject can be performed directly and/or indirectly.

However, alternatively and/or additionally, treating of a subject could comprise treating at least one of heart, cardiac muscle, muscle, smooth muscles, nerves, Renin Angiotensin system, kidney, adrenal gland and/or sinoatrial/atrioventricular nodes.

In this embodiment, the neuromodulation system is a neurostimulation system, wherein the neurostimulation system is or comprises an electrical neurostimulation system.

However, alternatively and/or additionally, a mechanical neurostimulation system and/or pharmacological neurostimulation system could be generally possible.

In this embodiment, the neurostimulation system is an epidural electrical stimulation system.

In this embodiment, the epidural electrical stimulation system is used to provide stimulation at least partially to the level of T10-L1 of the subject.

In an alternative embodiment, the stimulation system could be alternatively and/or additionally at least one of a transcutaneous electrical stimulation system, functional electrical stimulation system, intramuscular stimulation system, dorsal root ganglion stimulation system, subdural stimulation system, cardiac stimulation system, optogenetics, optotrodes, patch-clamp, intra-cellular electrodes and/or extra-cellular electrodes.

An electrical stimulation system (e.g. epidural and/or subdural stimulation system) could generally be used to provide stimulation at least partially to the level of T6 or higher and/or T1-T6 of the subject.

For treating cardiac failure and/or spinal cord injury, an electrical stimulation system (e.g. epidural and/or subdural stimulation system) could be used to provide stimulation at least partially to the level of T1-T5 of the subject.

For treating hypertonia and/or spinal cord injury (e.g. when autonomic dysreflexia occurs) the electrical stimulation system (e.g. epidural and/or subdural stimulation system) could be used to provide stimulation at least partially at to level of C1-C4 of the subject and above.

However, alternatively and/or additionally, the neurostimulation system could be a dorsal root ganglion stimulation system and/or subdural stimulation system and/or cardiac stimulation system.

The use of the neuromodulation system according to this embodiment comprises the use of the neuromodulation system post-implantation of the subject, in particular post implantation of the epidural electrical stimulation system.

In general, the use of the neuromodulation system could comprise the use of the neuromodulation system throughout rehabilitation of the subject and/or during at least one stage of rehabilitation.

In particular, the stage of rehabilitation may be or may comprise at least one of stabilization in bed, mobilization in bed, mobilization at bedside, verticalization, blood pressure lowering, blood pressure rehabilitation, blood pressure stabilization, cardiac rehabilitation, cardiac stabilization standing, stepping, balance training, locomotor training, fluent locomotion, pre-surgery, post-surgery, pre-implantation, post-setting of neuromodulation system, spinal shock and/or daily life activities.

In general, indoor and/or outdoor use of the neuromodulation system is possible in this embodiment.

Further, the use of the system is possible during activities performed by the subject.

In this embodiment, the use of the neuromodulation system is performed to modulate smooth muscle contraction (response).

However, alternatively and/or additionally, the use of the neuromodulation system could be performed to evoke responses such as but not limited to cardiac response, hormonal response, neurotransmitter response, sensory response and/or smooth muscle dilatation.

In this embodiment, the use of the neuromodulation system comprises the use of tonic neuromodulation, in particular, tonic neurostimulation.

In this embodiment, tonic neuromodulation may be applied with 1 to 125 Hz.

It could be possible that the system could provide frequency neuromodulation on top of a carrying frequency. In particular, the carrying frequency could be applied with 1 to 10 kHz, preferably 5 to 10 kHz.

However, alternatively and/or additionally, the use of burst and/or high-frequency neuromodulation, in particular neurostimulation could be generally possible.

In this embodiment, the neuromodulation system is an open-loop system.

In an alternative embodiment, a closed-loop system and/or pseudo open-loop system could be generally possible.

In general, a sensor of a closed-loop neuromodulation system could be or could comprise at least one of a tensiometer, pulse pressure measuring method, electrocardiogram, pulse oximetry, MRI, doppler ultrasound and/or any other type of sensor.

In general, the at least one sensor could provide quality and/or performance metrics of the use of the neuromodulation system.

The quality and/or performance metrics could be or could comprise cardiac parameters.

Alternatively, and/or additionally, the quality and/or performance metrics could comprise fatigue measures, stress measures (e.g. in body fluids), questionnaires (in particular QoL questionnaires), pulse pressure wave, blood O2 saturation, blood speed, QoL questionnaires, q and/or stress markers, e.g. in body fluids.

The use of the neuromodulation system in this embodiment could comprise the use of an assistive device.

However, alternatively, the use of the neuromodulation system in this embodiment could not comprise the use of an assistive device.

In general, the assistive device could be or could comprise at least one of pulling handles, cables from ceiling, aquatic equipment, a treadmill, an exoskeleton, an orthosis, a robot, a bike, a breather, crutches, a cuff, a cuff for functional electrical stimulation, a smartwatch, a motorized mattress, non-invasive ventilation, a standing frame, a rollator, a body weight support system, elastic bands, weights, a slide board, a step machine, a trike, a walker, a robot-physiotherapist, balls and/or half-balls.

In this embodiment, the neuromodulation system is used by a (treating) physician.

Alternatively, and/or additionally, the neuromodulation system could be used by another person.

The other person could be family member, a friend, a therapist, a physiotherapist, a nurse, an electrophysiologist, a non-professional user, a professional user, an occupational therapist, a surgeon and/or a technician (clinical/ FCE) of the subject.

In this embodiment, the use of the neuromodulation system comprises the use of a network device.

In particular, in this embodiment, the use of the neuromodulation system comprises the use of a smartphone.

Alternatively, and/or additionally, the use of the neuromodulation system could comprise the use of a network and/or platform and/or alternative network device.

In particular, the use of the neuromodulation system may comprise the use of at least one of a cloud, game console, internet, local database, local network, medical and/or rehabilitation device, smartwatch and/or social media.

In this embodiment, the use of the neurostimulation system comprises the use of a feedback system, wherein the feedback system is a visual feedback system.

In this embodiment, the feedback system comprises a screen.

Additionally, and/or alternatively, the feedback system could be or could comprise an audio feedback system and/or sensory-motoric feedback system.

Additionally, and/or alternatively, the feedback system could be or could comprise headphones and/or haptic signals.

A sensor may be at least one of a pressure sensor, rehabilitation device, electrode, electrode lead, electrocardiogram, magnetic resonance imaging, pulse oximetry sensor and/or pulse pressure sensors.

### Respiratory system

In a further preferred embodiment, the use of a neuromodulation system for treating a subject with at least one abnormal body function and/or disease comprises the treatment and/or modulation of at least one condition and/or function and/or dysfunction of the subject's respiratory system.

In this embodiment, the at least one condition and/or function and/or dysfunction is breathing deficiency.

However, in an alternative embodiment, the at least one condition and/or function and/or dysfunction could comprise at least one of bronchial tree obstruction and/or bronchial deficiency and/or alveolar deficiency.

In this embodiment, the at least abnormal body function is dyspnea.

In an alternative embodiment, the at least one abnormal body function and/or disease could be or could comprise at least one of chronic obstructive pulmonary disease, tumor, trauma, stroke, damaged dorsal root, spinal cord injury and/or amyotrophic lateral sclerosis (ALS).

In this embodiment, treating of the subject comprises treating the intercostal muscles of the subject.

However, alternatively and/or additionally, treating of a subject could comprise treating at least one of bronchia, smooth muscles of the pulmonary system, nerve, alveoli, muscles, solitary tract, pulmonary circulation and/or diaphragm.

In this embodiment, the neuromodulation system is a neurostimulation system, wherein the neurostimulation system is or comprises an electrical neurostimulation system.

However, alternatively and/or additionally, a mechanical neurostimulation system and/or pharmacological neurostimulation system could be generally possible.

In this embodiment, the neurostimulation system is an intramuscular stimulation system.

However, alternatively and/or additionally, the neurostimulation system could be a dorsal root ganglion stimulation system and/or subdural stimulation system and/or transcutaneous electrical stimulation system and/or epidural electrical stimulation system.

For treating dyspnea and/or spinal cord injury (e.g. for treating dyspnea after spinal cord injury) and/or chronic obstructive pulmonary disease, the electrical stimulation system (e.g. epidural and/or subdural stimulation system) could be used to provide stimulation at least partially to the level of C3-C5 of the subject.

For treating ALS (e.g. breathing deficiency caused by ALS), the electrical stimulation system (e.g. epidural and/or subdural stimulation system) could be used to provide stimulation at least partially to the level of T1-T6 of the subject.

In this embodiment, the use of the neuromodulation system comprises the use of the neuromodulation system post-implantation of the subject, in particular post implantation of the intramuscular stimulation system.

Alternatively, and/or additionally, the use of the neuromodulation system could comprise the use of the neuromodulation system throughout rehabilitation of the subject and/or during at least one stage of stabilization in bed, mobilization in bed, mobilization at bedside, verticalization, stabilization of blood pressure, standing, stepping, balance training, locomotor training, fluent locomotion, pre-surgery, post-surgery, pre-implantation, post-implantation, post-setting of neuromodulation system, spinal shock and/or daily life activities.

In this embodiment, the system is used indoor.

In particular, the indoor use comprises the use in least one of clinics, gym, physiotherapy room, occupational room, aquatic room, sport activity room, recreation facility, restaurant, cinema, bathroom, bedroom, kitchen, living room, etc.

However, in general, alternatively and/or additionally outdoor use of the neuromodulation system is possible.

Further, the use of the system is possible during activities performed by the subject.

The use of the neuromodulation system in this embodiment comprises the use of an assistive device.

In this embodiment, the assistive device is a breathing device.

Alternatively, and/or additionally, the assistive device could be an oxygenation device.

However, alternatively, the use of the neuromodulation system in this embodiment could not comprise the use of an assistive device.

In this embodiment, the use of the neuromodulation system comprises the use of tonic neuromodulation, in particular, tonic neurostimulation.

In this embodiment, tonic neuromodulation could be applied with 1 to 125 Hz.

It could be possible that the system could provide frequency neuromodulation on top of a carrying frequency. In particular, the carrying frequency could be applied with 1 to 10 kHz, preferably 5 to 10 kHz.

However, alternatively and/or additionally, the use of burst and/or high-frequency neuromodulation, in particular neurostimulation could be possible.

In this embodiment, the neuromodulation system may be a closed-loop system.

In an alternative embodiment, an open-loop system and/or pseudo open-loop system could be generally possible.

In this embodiment, the closed-loop system comprises a sensor.

In this embodiment, the sensor is a plethysmography sensor.

In an alternative embodiment, the system could comprise more than one sensor.

Alternatively, and/or additionally a sensor could be at least one of thermometer (e.g. a nose thermometer), a pressure sensor, pulse oximetry sensor, inertial measurement unit (IMU), goniometer, insole, electroencephalography sensor (EEG), electromyography sensor (EMG).

In general, the at least one sensor could provide quality and/or performance metrics of the use of the neuromodulation system.

In this embodiment, the quality and/or performance metric is respiratory rate.

Alternatively, and/or additionally, quality and/or performance metrics could comprise respiratory volume, blood O2 saturation and/or stress markers, e.g. in body fluids.

It is generally possible that the neuromodulation system is used and/or operated by at least one person.

In this embodiment, the neuromodulation system is used by the subject itself.

Alternatively, and/or additionally, the system could be used by a family member, a friend, a therapist, a physiotherapist, a nurse, an electrophysiologist, a non-professional user, a professional user, an occupational therapist, a surgeon, a technician (clinical/ FCE), and/or a (treating) physician of the subject.

In this embodiment, the use of the neuromodulation system is performed to evoke a response, in particular, respiration.

In an alternative embodiment, the use of the neuromodulation system could be performed to evoke additionally and/or alternatively cough.

### Sensory system

In a further preferred embodiment, the use of a neuromodulation system for treating a subject with at least one abnormal body function and/or disease comprises the treatment and/or modulation of at least one status and/or function and/or dysfunction of the subject's sensory system.

In this embodiment, the at least one condition and/or function and/or dysfunction comprises back pain.

In an alternative embodiment, the at least one condition and/or function and/or dysfunction could comprise odour and/or taste.

In this embodiment, the at least one abnormal body function and/or disease is trauma.

In an alternative embodiment, the at least one abnormal body function and/or disease could be or could comprise at least one of tumor, herniated disk, Parkinson's disease, Alzheimer's disease, neurodegenerative disease, stroke and/or spinal cord injury.

In this embodiment, treating of the subject comprises treating nerves of the subject.

In this embodiment, the treatment of the subject can be performed directly and/or indirectly.

In this embodiment, the neuromodulation system is a neurostimulation system, wherein the neurostimulation system is or comprises an electrical neurostimulation system.

However, alternatively and/or additionally, a mechanical neurostimulation system and/or pharmacological neurostimulation system could be generally possible.

In this embodiment, the neurostimulation system is an epidural electrical stimulation system.

In this embodiment, the epidural electrical stimulation system is used to provide stimulation at least partially to the level of T2-L1 of the subject.

In an alternative embodiment, the epidural electrical stimulation system could be used to provide stimulation at least partially above C1 of the subject.

In an alternative embodiment, the electrical neurostimulation system could be or could comprise at least one of transcutaneous electrical stimulation system, dorsal root ganglion stimulation system and/or subdural stimulation system.

The use of the neuromodulation system according to this embodiment comprises the use of the neuromodulation system post-implantation of the subject, in particular post implantation of the epidural electrical stimulation system.

Alternatively, and/or additionally, the use of the neuromodulation system could comprise the use of the neuromodulation system throughout rehabilitation of the subject and/or during at least one of stabilization in bed, mobilization in bed, mobilization at bedside, verticalization, blood pressure lowering, blood pressure rehabilitation, blood pressure stabilization, cardiac rehabilitation, cardiac stabilization, standing, stepping, balance training, locomotor training, fluent locomotion, pre-surgery, post-surgery, pre-implantation, post-setting of neuromodulation system, spinal shock and/or daily life activities.

In this embodiment, the neuromodulation system is an open-loop system.

However, alternatively, a closed-loop system and/or pseudo open-loop system could be generally possible.

In general, the closed-loop system could comprise a sensor.

In this embodiment, the use of the neuromodulation system comprises the use of burst neuromodulation, in particular, burst neurostimulation.

In general, burst neuromodulation could be applied by applying pairs, triplets, quadruplets, quintuplets of pulse at burst frequency of at least 200Hz, preferably 300 to 750 Hz, at an interburst frequency of 1 to 125 Hz, preferably 10 to 60Hz.

It could be possible that the system could provide frequency neuromodulation on top of a carrying frequency. In particular, the carrying frequency could be applied with 1 to 10 kHz, preferably 5 to 10 kHz.

However, alternatively and/or additionally, the use of tonic and/or high-frequency neuromodulation, in particular neurostimulation could be generally possible.

In this embodiment, the system is used indoor.

In this embodiment, the indoor use comprises the use in clinics.

Alternatively, and/or additionally, the indoor use could comprise at least one of gym, physiotherapy room, occupational room, aquatic room, sport activity room, recreation facility, restaurant, cinema, bathroom, bedroom, kitchen, living room, etc.

Alternatively, and/or additionally outdoor use of the neuromodulation system could be possible.

Further, the use of the system is generally possible during activities performed by the subject.

In this embodiment, the neuromodulation system is used and/or operated by at least one person.

In this embodiment, the neuromodulation system is used by the subject himself.

In general, any other person, such as a family member, a friend, a therapist, a physiotherapist, a nurse, an electrophysiologist, a non-professional user, a professional user, an occupational therapist, a surgeon, a technician (clinical/ FCE), and/or a (treating) physician of the subject could use the system.

### Locomotor system

In a further preferred embodiment, the use of a neuromodulation system for treating a subject with at least one abnormal body function and/or disease comprises the treatment and/or modulation of at least one status and/or function and/or dysfunction of the subject's locomotor system.

In this embodiment, the at least one condition and/or function and/or dysfunction comprises gait deficits.

In an alternative embodiment, the at least one condition and/or function and/or dysfunction could comprise individual limb paralysis, lower limb paralysis, upper limb paralysis, neck stabilization and/or trunk stabilization.

In this embodiment, the at least one abnormal body function and/or disease is spinal cord injury.

In an alternative embodiment, the at least one abnormal body function and/or disease could be or could comprise at least one of epilepsy, muscular hyperfatigability, herniated disk, tumor, amyotrophic lateral sclerosis, multiple sclerosis, trauma, stroke, damaged dorsal root, autoimmune disease, loss of sympathetic control, neurodegenerative disease, Parkinson's disease, essential tremor, Alzheimer's disease and/or osteoporosis.

In this embodiment, treating a subject comprises treating the spinal cord of the subject.

In an alternative embodiment, treating a subject could be or could comprise treating at least one of muscular cholinergic synapse, sympathetic and/or parasympathetic nervous system, spinal cord, dorsal root to muscle, muscle, smooth muscle and/or nerve.

In this embodiment, the neuromodulation system is used during locomotor training of the subject.

Alternatively and/or additionally, the neuromodulation system could be used throughout rehabilitation of the subject and/or during at least one of mobilization in bed, mobilization at bedside, verticalization, stepping, balance training, fluent locomotion, pre-surgery, post-surgery, pre-implantation, post-implantation, post-setting of neuromodulation system, spinal shock and/or daily life activities.

In this embodiment, the use of the neuromodulation system comprises the use of an assistive device.

In an alternative embodiment, the use of the neuromodulation system is combined with the use of an assistive device.

In this embodiment, the assistive device is an exoskeleton.

In an alternative embodiment, the assistive device may be or may comprise at least one of pulling handles, cables from ceiling, aquatic equipment, a treadmill, an orthosis, a robot, a bike, crutches, a cuff, a cuff for functional electrical stimulation, a smartwatch, a motorized mattress, a standing frame, a rollator, a body weight support system, elastic bands, weights, a slide board, a step machine, a trike, a walker, a robot-physiotherapist, balls and/or half-balls.

In this embodiment, the use of the neuromodulation system is performed to perform normal gait.

In an alternative embodiment, the task to be performed could additionally and/or alternatively comprise balancing, bending over, walking, climbing, climbing stairs, swimming and/or performing other tasks under water, sitting, sitting on ground, sit-to-stand, grasping, squatting, standing, inclined waking, neck training, posture training, normal neck posture, normal trunk posture, normal use of at least one affected limb, normal use of lower limbs, prevention of muscle weakness, training with body weight support and/or performing above-mentioned tasks with body weight support.

In this embodiment, the neuromodulation system is a neurostimulation system, wherein the neurostimulation system is or comprises an electrical neurostimulation system.

However, alternatively and/or additionally, a mechanical neurostimulation system and/or pharmacological neurostimulation system could be generally possible.

In this embodiment, the electrical neurostimulation system is an epidural electrical stimulation system.

In an alternative embodiment, the electrical neurostimulation system could be or could comprise at least one of transcutaneous electrical stimulation system, dorsal root ganglion stimulation system, intramuscular stimulation system and/or subdural stimulation system.

In this embodiment, the electrical stimulation system is used to provide stimulation at least partially to the level of L2-S1 of the subject.

In an alternative embodiment, the electrical stimulation system could be used to provide stimulation at least partially to the level of L2-S2 of the subject.

In an alternative embodiment, for the use of a neuromodulation system for neck stabilization, the electrical stimulation system could be used to provide stimulation at least partially to the level of T2-L1, preferably to the level of T7-L1 of the subject.

In an alternative embodiment, for the use of a neuromodulation system for treating upper and/or lower limb paralysis, the electrical stimulation system could be used to provide stimulation at least partially to the level of C1-S1 of the subject.

In this embodiment, the neuromodulation system is a closed-loop system.

However, alternatively, an open-loop system and/or pseudo open-loop system could be generally possible.

In this embodiment, the closed-loop system comprises a sensor.

In this embodiment, the sensor is an IMU.

Alternatively, and/or additionally, the sensor could be or could comprise at least one of an accelerometer, pressure sensor, motion capture sensor, force plate, rehabilitation device, electrode, electrode lead, infrared camera, GPS, smartphone, smartwatch, action-camcorder, video processing, virtual reality module, magnetic resonance imaging, goniometer, insole, EEG, skin resistance sensor and/or infrared sensor.

In this embodiment, the use of the neuromodulation system comprises the use of tonic neuromodulation, in particular tonic neurostimulation.

In this embodiment, tonic neuromodulation could be applied with 1 to 125 Hz.

It could be possible that the system could provide frequency neuromodulation on top of a carrying frequency. In particular, the carrying frequency could be applied with 1 to 10 kHz, preferably 5 to 10 kHz.

However, alternatively and/or additionally, the use of burst and/or high-frequency neuromodulation, in particular neurostimulation could be generally possible.

In this embodiment, the use of the neuromodulation system comprises the use of the neuromodulation system post-implantation of the neuromodulation system.

Alternatively, and/or additionally, the use of the neuromodulation system could comprise the use of the neuromodulation system throughout rehabilitation of the subject and/or during at least one of stabilization in bed, mobilization in bed, mobilization at bedside, standing, stepping, balance training, locomotor training, fluent locomotion, pre-surgery, post-surgery, post-setting of neuromodulation system, spinal shock and/or daily life activities.

In this embodiment, the system is used indoor.

In this embodiment, the indoor use comprises the use in clinics.

Alternatively, and/or additionally, the indoor use may comprise at least one of gym, physiotherapy room, occupational room, aquatic room, sport activity room, recreation facility, restaurant, cinema, bathroom, bedroom, kitchen, living room, etc.

Alternatively, and/or additionally outdoor use of the neuromodulation system could be possible.

Further, the use of the system is generally possible during activities performed by the subject.

In this embodiment, the neuromodulation system is used and/or operated by at least one person.

In this embodiment, the neuromodulation system is used by the subject himself.

In general, any other person, such as a family member, a friend, a therapist, a physiotherapist, a nurse, an electrophysiologist, a non-professional user, a professional user, an occupational therapist, a surgeon, a technician (clinical/ FCE), and/or a (treating) physician of the subject could the neuromodulation system.

### Thermoregulation system

In a further preferred embodiment, the use of a neuromodulation system for treating a subject with at least one abnormal body function and/or disease comprises the treatment and/or modulation of at least one status and/or function and/or dysfunction of the subject's thermoregulation system.

In this embodiment, the at least one condition and/or function and/or dysfunction comprises cutaneous vascularization.

In an alternative embodiment, the at least one condition could additionally and/or alternatively comprise at least one of blood volume, thermoregulation, lipogenesis/lipolysis imbalance, sweat glands and/or lipid metabolism.

In this embodiment, the at least one abnormal body function and/or disease is spinal cord injury.

In an alternative embodiment, the at least one abnormal body function and/or disease could be or could comprise at least one of tumor, obesity, trauma, stroke, damaged dorsal root, and/or hyperthyroidism.

In this embodiment, treating a subject comprises treating the spinal cord of the subject.

In an alternative embodiment, treating the subject could be or could comprise treating at least one of sympathetic and/or parasympathetic nervous system, dorsal root to muscle, muscle, smooth muscle and/or nerve, preferably in the brain, e.g. in the thalamus and/or hypothalamus of the subject.

In this embodiment, the neuromodulation system is used during spinal shock.

Alternatively and/or additionally, the neuromodulation system could be used throughout rehabilitation of the subject and/or during at least one of stabilization in bed, mobilization in bed, mobilization at bedside, verticalization, blood pressure lowering, blood pressure rehabilitation, blood pressure stabilization, cardiac rehabilitation, cardiac stabilization, standing, stepping, balance training, locomotor training, fluent locomotion, pre-surgery, post-surgery, pre-implantation, post-implantation, post-setting of neuromodulation system and/or daily life activities.

In general, the use of the neuromodulation system could comprise the use of an assistive device.

In this embodiment, the neuromodulation system is a neurostimulation system, wherein the neurostimulation system is an electrical neurostimulation system.

However, alternatively and/or additionally, a mechanical neurostimulation system and/or pharmacological neurostimulation system could be generally possible.

In this embodiment, the electrical neurostimulation system is an epidural electrical stimulation system.

In an alternative embodiment, the electrical neurostimulation system could be or could comprise at least one of transcutaneous electrical stimulation system, dorsal root ganglion stimulation system, intramuscular stimulation system and/or subdural stimulation system.

In this embodiment, the electrical stimulation system is used to provide stimulation at least partially to the level of T7 of the subject.

In an alternative embodiment, an electrical stimulation system could be used to provide stimulation at least partially to the brain of the subject, preferably to the hypothalamus of the subject.

In this embodiment, the neuromodulation system is a closed-loop system.

However, alternatively, an open-loop system and/or pseudo open-loop system could be generally possible.

In this embodiment, the closed-loop system comprises a sensor.

In this embodiment, the sensor is a thermometer (in particular a skin thermometer).

Alternatively, and/or additionally, the sensor could be or could comprise at least one of an electrode, electrode lead, implanted thermometer and/or other type of sensor.

In this embodiment, the use of the neuromodulation system comprises the use of burst neuromodulation, in particular, burst neurostimulation.

In general, burst neuromodulation could be applied by applying pairs, triplets, quadruplets, quintuplets of pulse at burst frequency of at least 200Hz, preferably 300 to 750 Hz, at an interburst frequency of 1 to 125 Hz, preferably 10 to 60Hz.

However, alternatively and/or additionally, the use of tonic and/or high-frequency neuromodulation, in particular neurostimulation could be generally possible.

In this embodiment, the system is used indoor.

In this embodiment, the indoor use comprises the use in clinics.

Alternatively, and/or additionally, the indoor use may comprise at least one of gym, physiotherapy room, occupational room, aquatic room, sport activity room, recreation facility, restaurant, cinema, bathroom, bedroom, kitchen, living room, etc.

Alternatively, and/or additionally outdoor use of the neuromodulation system could be possible.

Further, the use of the system is generally possible during activities performed by the subject.

In this embodiment, the neuromodulation system is used and/or operated by at least one person.

In this embodiment, the neuromodulation system is used by the subject himself.

In general, any other person, such a family member, a friend, a therapist, a physiotherapist, a nurse, an electrophysiologist, a non-professional user, a professional user, an occupational therapist, a surgeon, a technician (clinical/ FCE), and/or a (treating) physician of the subject could use the neuromodulation system.

## Claims

1. The use of a neuromodulation system for treating a subject with at least one abnormal body function and/or disease comprising the treatment and/or modulation of at least one condition and/or function and/or dysfunction of the subject's gastrointestinal system, cardiovascular system, sensory system, urinary system, respiratory system, reproductive system, thermoregulation system and/or locomotor system.

2. The use of a neuromodulation system according to claim 1, **characterized in that** the at least one condition and/or function and/or dysfunction comprises at least one of acetylcholine dysregulation, acidity of stomach, autonomic dysreflexia, back pain, sphincter function, blood volume dysregulation, breathing deficiency, bronchial tree obstruction, cardiac activity, cholesterol imbalance, motor deficits, mucosa renewal, immune system deficit, immobility of the stomach and/or intestine and/or colon, lipogenesis/lipolysis imbalance, lower limb paralysis and/or upper limb paralysis (bilateral and/or unilateral), detrusor muscle, bronchial deficiency, alveolar deficiency, mean venous pressure, mean arterial pressure, mucous layer of stomach, erectile dysfunction, neck stabilization, odour, hypotension, orthostatic hypotension, hypertension, salivary glands function, supraspinal cardiovascular regulation, swallowing, taste, thermoregulation, trunk stabilization, vision, cutaneous vascularization, sweat glands, lipid metabolism and/or glycogen metabolism.

3. The use of a neuromodulation system according to claim 1 or claim 2, **characterized in that** the at least one abnormal body function and/or disease is or comprises myasthenia, dystonia, epilepsy, muscular hyperfatigability, ulcers, Helicobacter Pylori infection, inflammatory bowel disease, Crohn's disease, ulcerative colitis, irritable bowel syndrome, spinal cord injury, incontinence, hypertension, hypotension, sudden episodic high blood pressure, dyspnea, chronic obstructive pulmonary disease, cardiac failure, cardiac dysrhythmia, bradycardia, tachycardia, cardiac arrest, impairment of genital plexus (e.g. after prostate removal), HIV infection, herniated disk, tumor, obesity, gastroesophageal reflux disease, lower esophageal sphincter failure, achalasia, amyotrophic lateral sclerosis, trauma, stroke, damaged dorsal root, pernicious anemia, multiple sclerosis, diabetes mellitus, autoimmune disease, Sjogren syndrome, Mikulicz syndrome, loss of sympathetic control, dysphagia, overreactive bladder syndrome, neurodegenerative disease, Parkinson's disease, Alzheimer's disease, essential tremor, schizophrenia and/or osteoporosis.

4. The use of a neuromodulation system according to one of the preceding claims, **characterized in that** treating a subject is or comprises treating of at least one of muscular cholinergic synapse, stomach, sympathetic and/or parasympathetic nervous system, heart, cardiac muscle, spinal cord, lower urinary sphincter, autonomous and/or voluntary regulated sphincters, Renin Angiotensin system, kidney, lower bowel sphincter, diaphragm, intercostal muscle, bronchia, smooth muscles of the pulmonary system, solitary tract, sinoatrial/atrioventricular nodes, pancreas, genital plexus, dorsal root to muscle (e.g. pharynx), colon, vago-vagal reflex, cajal cell, muscle, smooth muscle, nerve, concerned nerve or group of nerves, concerned plexus (including but not limited to genital, Auerbach, Meissner, cardiac, pulmonary, cervical, lombal, sacral and/or coeliaque plexus), liver, adrenal gland, lower esophageal sphincter, alveoli, pulmonary circulation, associated cortex and/or regulatory center, salivary gland and/or detrusor muscle.

5. The use of the neuromodulation system according to one of the preceding claims throughout rehabilitation of the subject and/or during at least one stage of rehabilitation, **characterized in that** the stage of rehabilitation is or comprises at least one of stabilization in bed, mobilization in bed, mobilization at bedside, verticalization, blood pressure lowering, blood pressure rehabilitation, blood pressure stabilization, cardiac rehabilitation, cardiac stabilization, standing, stepping, balance training, locomotor training, fluent locomotion, pre-surgery, post-surgery, pre-implantation, post-implantation, post-setting of neuromodulation system, spinal shock and/or daily life activities.

6. The use of the neuromodulation system according one of the preceding claims, **characterized in that** the use of the neuromodulation system comprises or is combined with the use of an assistive device, wherein the assistive device is or comprises at least one of a breathing device, pulling handles, cables from ceiling, aquatic equipment, a treadmill, an exoskeleton, an orthosis, a robot, a bike, a breather, crutches, a cuff, a cuff for functional electrical stimulation, a smartwatch, a motorized mattress, non-invasive ventilation, a standing frame, a rollator, body-weight support system, elastic bands, weights, a slide board, a step machine, a trike, a walker, a robot-physiotherapist, balls and/or half-balls.

7. The use of a neuromodulation system according to one of the preceding claims, **characterized in that** the neuromodulation system is a neurostimulation system, wherein the neurostimulation system is or comprises an electrical neurostimulation system and/or mechanical neurostimulation system and/or pharmacological neurostimulation system.

8. The use of neuromodulation system according to claim 7, **characterized in that** the electrical neurostimulation system is at least one of a transcutaneous electrical stimulation system, epidural electrical stimulation system, functional electrical stimulation system, intramuscular stimulation system, dorsal root ganglion stimulation system, subdural stimulation system, cardiac stimulation system, optogenetics, optotrodes, patch-clamp, intra-cellular electrodes and/or extra-cellular electrodes.

9. The use of a neurostimulation system according to claim 7 or claim 8, **characterized in that** the system is an open-loop system and/or closed-loop system and/or pseudo open-loop system.

10. The use of a neurostimulation system according to claim 9, **characterized in that** the closed-loop system comprises at least one sensor.

11. The use of a neurostimulation system according to one of the preceding claims, **characterized in that** the use of the neurostimulation system comprises and/or is combined with the use of a feedback system, wherein the feedback system is an audio and/or visual and/or sensory-motoric feedback system.

12. The use of a system for providing neuromodulation according to one of the preceding claims, **characterized in that** the system provides tonic and/or burst and/or high-frequency neuromodulation.

13. The use of a system for providing neuromodulation according to one of the preceding claims, **characterized in that** the system provides frequency neuromodulation on top of a carrying frequency.
